(19) Europäisches Patentamt
European Patent Office
Office européen des brevets

(11) **EP 4 088 779 A1**

(12) **EUROPÄISCHE PATENTANMELDUNG**

(43) Veröffentlichungstag:
**16.11.2022   Patentblatt 2022/46**

(21) Anmeldenummer: **21173008.0**

(22) Anmeldetag: **10.05.2021**

(51) Internationale Patentklassifikation (IPC):
***A61N 5/06*** *(2006.01)*

(52) Gemeinsame Patentklassifikation (CPC):
**A61N 5/0614;** A61N 2005/0615; A61N 2005/0661

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Schaffron, Günter**
**49716 Meppen (DE)**

(72) Erfinder: **Schaffron, Günter**
**49716 Meppen (DE)**

(74) Vertreter: **Hauck Patentanwaltspartnerschaft mbB**
**Postfach 11 31 53**
**20431 Hamburg (DE)**

(54) **STRAHLUNGSQUELLE UND HAUTBESTRAHLUNGSGERÄT**

(57) Strahlungsquelle zur Abgabe von UV-Strahlung für ein Hautbestrahlungsgerät zur Bräunung der Haut, gekennzeichnet durch ein Verhältnis von NMSC-wirksamer Bestrahlungsstärke ($E_{NMSC}$) zu Erythem-wirksamer Bestrahlungsstärke ($E_{er}$) von kleiner oder gleich 0,9 ($E_{NMSC}/E_{er} \leq 0{,}9$).

Fig. 1

EP 4 088 779 A1

**Beschreibung**

Die Erfindung betrifft eine Strahlungsquelle zur Abgabe von UV-Strahlung zur Bräunung der Haut, sowie ein Hautbestrahlungsgerät umfassend eine solche Strahlungsquelle.

Als Strahlungsquellen für Hautbestrahlungsgeräte zur Bräunung der Haut, also für Solarien, werden üblicherweise UV-Niederdruckröhren oder UV-Hochdruckbrenner mit Filterscheiben eingesetzt. So können beispielsweise lediglich UV-Niederdruckröhren oder UV-Hochdruckbrenner als Bestrahlungsquellen vorgesehen sein. Auch kann eine Kombination aus UV-Niederdruckröhren und UV-Hochdruckbrennern mit Filterscheiben vorgesehen sein, wobei üblicherweise die Niederdruckröhren zur Bestrahlung des Körperbereichs und der Hochdruckbrenner zur Bestrahlung des Gesichtsbereichs verwendet werden. Auch können LED-Strahler für den Gesichtsbereich oder sogar zur Ganzkörperbestrahlung eingesetzt werden.

Strahlungsquellen zur Bräunung der Haut geben UV-Strahlung üblicherweise in einem Bereich zwischen 280 und 400 nm oder in Teilbereichen davon ab. Strahlung im UVA-Bereich zwischen 315 und 400 nm Wellenlänge hat eine größere Eindringtiefe in biologisches Gewebe und trägt im Wesentlichen zur direkten Pigmentierung bei, also zu der Entwicklung einer kurzfristigen Bräune. UVB-Strahlung hingegen, im Wellenlängenbereich von 280 bis 315 nm, dringt weniger tief in biologisches Gewebe ein und bewirkt dadurch eine verzögerte Bildung von Melanin und damit eine indirekte Pigmentierung, also eine verzögerte, längerfristige Bräunung. Um ein optimales Bräunungsergebnis zu erhalten, ist ein abgestimmtes Verhältnis von UVA- zu UVB-Strahlung und eine definierte Bestrahlungsstärke von Bedeutung.

Bei der biologischen Bestrahlungsstärke handelt es sich um die Intensität der Strahlung, die auf der menschlichen Haut eine entsprechende Wirkung erzielt. Hierbei sind neben der bereits erläuterten direkten bzw. indirekten Pigmentierung auch die erythemwirksame sowie die in NMSC-wirksame Bestrahlungsstärke von Relevanz. Die erythemwirksame Strahlung ist für die Bildung von Sonnenbrand verantwortlich, sodass die erythemwirksame Bestrahlungsstärke im Wesentlichen die Besonnungszeit bestimmt. Der Hauptteil der erythemwirksamen Strahlung liegt im UVB-Bereich von 280 bis 315 nm.

Die indirekte Pigmentierung und damit die gewünschte längerfristige Bräunung der Haut steht in direktem Zusammenhang mit der erythemwirksamen Bestrahlungsstärke, da diese im Wesentlichen ebenfalls im UVB-Bereich stattfindet. Je höher die für die indirekte Pigmentierung verantwortliche Bestrahlungsstärke, desto höher also die erythemwirksame Bestrahlungsstärke und damit die Sonnenbrandgefahr.

NMSC-Strahlung bezeichnet den Teil der UV-Strahlung, der für die Bildung von Nicht-Melanom-Hautkrebs verantwortlich ist (NMSC = *non melanoma skin cancer*). Der Hauptteil dieser Strahlung liegt im Wellenlängenbereich von 280 bis 340 nm und damit in vergleichbaren Bereichen wie die erythemwirksame und die für die indirekte Pigmentierung verantwortliche Strahlung. Da die NMSC-Strahlung Strahlung krebserregend wirken kann, ist eine Reduzierung derselben wünschenswert.

Der Erfindung liegt die Aufgabe zugrunde, eine Strahlungsquelle sowie ein Bestrahlungsgerät zur Verfügung zu stellen, die bei ausreichender Bräunungswirkung ein geringeres NMSC-Krebsrisiko darstellen.

Die Aufgabe wird gelöst durch eine Strahlungsquelle gemäß Anspruch 1, sowie ein Hautbestrahlungsgerät gemäß Anspruch 10. Vorteilhafte Ausgestaltungen sind Gegenstand der abhängigen Ansprüche, der Beschreibung sowie der Figuren.

Die erfindungsgemäße Strahlungsquelle zur Abgabe von UV-Strahlung für ein Hautbestrahlungsgerät zur Bräunung der Haut weist ein Verhältnis von NMSC-wirksamer Strahlungsstärke $E_{NMSC}$ zu erythemwirksamer Bestrahlungsstärke $E_{er}$ von kleiner oder gleich 0,9 auf, es gilt also $E_{NMSC}/E_{ER} \leq 0,9$.

Die Strahlungsquelle ist zur Abgabe von UV-Strahlung ausgebildet, insbesondere zur Abgabe von UV-Strahlung im Wellenlängenbereich von 280 bis 400 nm bzw. 280 bis 340 nm. Die Strahlungsquelle kann hierfür ein oder mehrere UV-Strahlung abgebende Strahlungskörper umfassen, die entsprechend ausgebildet oder angesteuert sein können. Beispielsweise kann die Strahlungsquelle ein oder mehrere UV-Niederdruckröhren mit einem Gasgemisch, wie einer Phosphormischung, umfassen. Durch entsprechende Zusammenstellung des Gasgemischs kann das erfindungsgemäße Verhältnis erreicht werden. Auch kann alternativ oder zusätzlich eine Filterscheibe vorgesehen sein, insbesondere bei Hochdruckbrennern, um von dem UV-Strahlungskörper abgegebene Strahlung in gewissen Wellenlängenbereichen weg zu filtern. Auch somit kann das erfindungsgemäße Verhältnis erreichbar sein. Insbesondere kann das erfindungsgemäße Verhältnis erreicht werden durch eine entsprechende Gas-/Phosphormischung sowie eine entsprechende Wahl des Glases der Filterscheibe.

Die Strahlungsquelle kann eine Steuereinheit umfassen zur Ansteuerung des oder der Strahlungskörper der Strahlungsquelle. Die Strahlungsquelle ist dazu ausgebildet, das erfindungsgemäße Verhältnis der Bestrahlungsstärken abzugeben, entweder durch direkte Erzeugung der entsprechenden Strahlung über die Strahlungskörper und/oder durch Filtern einzelner Wellenlängenbereiche der von den Strahlungskörpern erzeugten Strahlung, sodass auf der Haut lediglich eine Bestrahlungsstärke im genannten Verhältnis ankommt. Die Strahlungsquelle kann also derart ausgebildet sein, dass sie bzw. ihre Strahlungskörper in Wellenlängenbereichen, die ein anderes Verhältnis der Bestrahlungsstärken zur Folge hätten, keine Strahlung aussendet.

Der Erfinder hat festgestellt, dass eine Strahlungsquelle mit dem erfindungsgemäßen Verhältnis von NMSC-wirksamer Bestrahlungsstärke zu erythemwirksamer Bestrahlungsstärke ein gutes Bräunungsergebnis bei gleichzeitig verringertem Hautkrebsrisiko hervorbringt. Insbesondere wird somit eine Verringerung der NMSC-wirksamen Bestrahlungsstärke gegenüber den Strahlungsquellen bekannter Solarien ermöglicht. Hierfür kann entsprechend die gesamte wirksame Bestrahlungsstärke reduziert sein. Beispielsweise kann somit die NMSC-wirksame Bestrahlungsstärke auf einen Wert von $\leq 0{,}20$ W/m$^2$ begrenzt werden und damit insbesondere niedriger sein als die NMSC-wirksame Bestrahlungsstärke der natürlichen Sonne im Hochsommer in deutschen Breitengraden. Mit dem erfindungsgemäßen Verhältnis kann, bei entsprechender Bestrahlungsleistung, eine gewünschte Bräunungswirkung besonders schnell erreicht werden. Somit kann die Besonnungszeit verringert werden, was die NMSC-Belastung und damit das Hautkrebsrisiko stark reduziert. Entsprechende Solarien können damit der natürlichen Besonnung vorzuziehen sein.

Nach einer Ausgestaltung ist das Verhältnis von NMSC-wirksamer Bestrahlungsstärke zu erythemwirksamer Bestrahlungsstärke im Wesentlichen über den gesamten für die Hautbräunung relevanten Wellenlängenbereich kleiner oder gleich 0,9.

Dies kann nach einer Ausgestaltung ein Wellenlängenbereich im Wesentlichen von ca. 280 nm bis ca. 400 nm sein, insbesondere im Wesentlichen von ca. 280 nm bis ca. 340 nm. Es kann somit insbesondere über den gesamten Wellenlängenbereich, der in Solarien üblicherweise auftritt und für die Bräunung der Haut verantwortlich ist, eine Reduzierung der NMSC-wirksamen Bestrahlungsstärke ermöglicht werden. Der Begriff "im Wesentlichen" ist dabei insbesondere derart zu verstehen, dass innerhalb der genannten Wellenlängenbereiche zumindest ein enger Wellenlängenbereich von bevorzugt nicht mehr als 5 nm oder 6 nm vorliegen kann, innerhalb dessen das erfindungsgemäße Verhältnis nicht erreicht wird. Es kann sich insbesondere um den später noch im Detail erläuterten Quecksilberpeak handeln.

Wie bereits angesprochen, kann das erfindungsgemäße Verhältnis der Bestrahlungsstärken erreicht werden durch entsprechende Anpassung der Strahlungsquelle bzw. der Strahlungskörper der Strahlungsquelle. Nach einer Ausgestaltung sendet die Strahlungsquelle in einem Wellenlängenbereich von ca. 280 nm bis ca. 340 nm, insbesondere in einem Wellenlängenbereich von ca. 296 nm bis ca. 333 nm keine Strahlung aus. Dies kann, wie ebenfalls angesprochen, erreicht werden, indem entweder eine Strahlungsquelle gewählt wird, deren Strahlungskörper in den genannten Wellenlängenbereichen keine Strahlung erzeugen oder in dem eine Filterscheibe vor die Strahlungsquelle gesetzt wird, die in den entsprechenden Wellenlängenbereichen Strahlung wegfiltert. Hierdurch kann das erfindungsgemäße Verhältnis von NMSC-wirksamer Bestrahlungsstärke zu erythemwirksamer Bestrahlungsstärke erreicht werden. Die Angabe "ca." ist dabei insbesondere als eine Schwankung von +/- 5 nm um den jeweiligen Wert zu verstehen. Wie der Erfinder festgestellt hat, kann durch eine solche Anpassung der emittierten Strahlung das erfindungsgemäße Verhältnis erzielt werden und damit bei ausreichender Bräunungsleistung das NMSC-Krebsrisiko reduziert werden.

Nach einer Ausgestaltung ist die Strahlungsquelle dazu ausgebildet, oberhalb oder ab einer Wellenlänge von 340 nm aufwärts keine Strahlung abzugeben. Der Strahlungskörper kann entsprechend ausgebildet sein, also keine Strahlung oberhalb von 340 nm erzeugen. Alternativ kann von dem Strahlungskörper eventuell oberhalb von 340 nm erzeugte Strahlung weggefiltert werden, bevor diese eine zu bräunende Person erreicht. Durch eine solche Begrenzung des Wellenlängenbereichs nach oben kann die Bestrahlung auf den für die Bräunung besonders relevanten Bereich begrenzt werden.

Nach einer Ausgestaltung weist die Strahlungsquelle ein Verhältnis von Vitamin-D3-wirksamer Bestrahlungsstärke $E_{D3}$ zu erythemwirksamer Bestrahlungsstärke $E_{er}$ von mindestens 0,14 auf. In anderen Worten kann die Vitamin-D3-wirksame Bestrahlungsstärke mindestens 14% der erythemwirksamen Bestrahlungsstärke betragen. Nach einer Ausgestaltung beträgt die Vitamin-D3-wirksame Bestrahlungsstärke $E_{D3}$ mindestens 0,03 Watt pro Quadratmeter.

Nach einer Ausgestaltung umfasst die Strahlungsquelle eine UV-Leuchtstofflampe als Strahlungskörper, insbesondere eine UV-Niederdrucklampe. Die Strahlungsquelle kann mehrere UV-Leuchtstofflampen umfassen. Als UV-Leuchtstofflampe kann nach einer weiteren Ausgestaltung beispielsweise eine UV-Niederdrucklampe und/oder ein UV-Hochdruckbrenner, insbesondere mit Filterscheibe, oder auch mehrere vorgesehen sein. Durch exakte Zusammenstellung des in den Lampen enthaltenen Leuchtstoffs kann das erfindungsgemäße Verhältnis erreicht werden, insbesondere ein Abstrahlen in den erwähnten Wellenlängenbereichen vermieden werden. Als Leuchtstoff kann beispielsweise eine Phosphormischung zum Tragen kommen. Bei UV-Hochdruckbrennern wird das erfindungsgemäße Verhältnis hingegen mit der Kombination aus Hochdruckbrenner und Filterscheibe(n) erreicht.

Nach einer diesbezüglichen Ausgestaltung ist die UV-Niederdrucklampe dazu ausgebildet, in einem Wellenlängenbereich von ca. 296 nm bis ca. 333 nm nur in einem den Quecksilberpeak betreffenden Wellenlängenbereich Strahlung auszusenden. Der den Quecksilberpeak betreffende Wellenlängenbereich kann sich insbesondere von ca. 311 nm bis ca. 315 nm erstrecken. In anderen Worten kann die UV-Niederdrucklampe dazu ausgebildet sein, in einem Wellenlängenbereich von ca. 296 nm bis ca. 310 nm und/oder in einem Wellenlängenbereich von ca. 316 nm bis ca. 333 nm keine Strahlung auszusenden. Wie erwähnt, wird bevorzugt in einem Wellenlängenbereich von ca. 280 nm bis ca. 340 nm, besser in einem Wellenlängenbereich von ca. 296 nm bis ca. 333 nm keine Strahlung ausgesendet. So kann vollständig über die genannten Wellenlängenbereiche das erfindungsgemäße Verhältnis von NMSC-wirksamer Bestrahlungsstärke zu erythemwirksamer Bestrahlungsstärke erreicht werden. Bei UV-Niederdrucklampen als Strahlungsquellen kommt es jedoch zum sogenannten Quecksilberpeak innerhalb der genannten Wellenlängenbereiche, insbesondere zwischen 311 nm und 315 nm. Dieser Peak lässt sich auch durch Feineinstellung der Gas-/Phosphormischung nicht vollständig eliminieren. Innerhalb des genannten Wellenlängenbereiches des Quecksilberpeaks kann folglich ein anderweitiges Verhältnis von NMSC-wirksamer Bestrahlungsstärke zu erythemwirksamer Bestrahlungsstärke vorliegen, insbesondere ein Verhältnis von > 1,0.

Nach einer Ausgestaltung umfasst die Strahlungsquelle zumindest eine UV-LED (*light emitting diode*, Leuchtdiode) als Strahlungskörper. Alternativ oder zusätzlich zu UV-Leuchtstofflampen können folglich auch UV-LEDs als Strahlungskörper der Strahlungsquelle vorgesehen sein. Beispielsweise kann eine Kombination aus UV-Niederdruckröhren und UV-LEDs vorgesehen sein, wobei anstatt der wie eingangs erläutert üblicherweise vorgesehenen UV-Hochdruckbrenner UV-LEDs als alleinige Strahlungskörper im Gesichtsbereich eingesetzt werden können, während die UV-Niederdruckröhren für den Körperbereich zuständig sind. Auch können grundsätzlich lediglich UV-LEDs als Strahlungskörper in Solarien vorgesehen sein. Die UV-LEDs können dazu ausgebildet sein, lediglich UV-Licht oder auch zusätzlich sichtbares Licht abzugeben. Insbesondere können ergänzend LEDs vorgesehen sein, die sichtbares Licht abgeben, um dem Benutzer zu verdeutlichen, dass die Bestrahlungsquelle eingeschaltet ist. LEDs können zielgenau zur Abgabe von Strahlung in bestimmten Wellenlängenbereichen ausgebildet sein. Dies kann durch eine entsprechende Materialwahl der indirekten bzw. direkten Halbleiter der LED erfolgen. Somit kann eine Strahlungsquelle mit UV-LEDs durch entsprechende Materialwahl das erfindungsgemäße Verhältnis von NMSC-wirksamer Bestrahlungsstärke zu erythemwirksamer Bestrahlungsstärke erreichen, insbesondere also in Wellenlängenbereichen, die ein anderes Verhältnis zur Folge hätten, keine Strahlung aussenden.

Nach einer Ausgestaltung erzeugt die Strahlungsquelle eine Bestrahlungsstärke für die direkte Pigmentierung, $E_{pi}$, von mindestens 180 W/m², bevorzugt von mindestens 200 W/m². In anderen Worten ist die Strahlungsquelle dazu ausgebildet, im Wellenlängenbereich der direkten Pigmentierung eine Bestrahlungsstärke von 180 W/m² bzw. 200 W/m² oder mehr abzugeben. Die Strahlungsquelle kann entsprechend angesteuert werden. Es kann somit eine gute Bräunung erreicht, weiterhin jedoch das erfindungsgemäße Verhältnis der Bestrahlungsstärken eingehalten werden.

Die Erfindung betrifft zudem ein Hautbestrahlungsgerät zur Bräunung der Haut umfassend mindestens eine solche Strahlungsquelle. Das Hautbestrahlungsgerät kann mehrere Strahlungsquellen dieser Art umfassen, insbesondere Strahlungsquellen unterschiedlicher Ausbildung, wie bereits angesprochen. Das Hautbestrahlungsgerät ist insbesondere ein Solarium, wie eine Sonnenbank oder eine Sonnendusche.

Ein Ausführungsbeispiel der Erfindung wird im Folgenden anhand von Figuren erläutert. Es zeigen:

Figur 1　　ein Diagramm mit den Wirkungsfunktionen für das Erythem und den Nicht-Melanom-Hautkrebs (NMSC),

Fig. 2a　　die Bestrahlungsstärke vierer unterschiedlicher Anlagen, und

Fig. 2b　　die Bestrahlungsdosis für die vier Anlagen.

Die gesamte biologisch wirksame Bestrahlungsstärke wird bekanntermaßen bestimmt durch:

$$E_{\text{eff}} = \sum_{250\,\text{nm}}^{400\,\text{nm}} S_\lambda E_\lambda \Delta_\lambda \,,$$

wobei $E_{\text{eff}}$ die gesamte wirksame Bestrahlungsstärke, $S_\lambda$ die relative spektrale Wirksamkeit (Wichtungsfaktor), $E_\lambda$ die spektrale Bestrahlungsstärke in W/m², sowie $\Delta_\lambda$ das Intervall der Wellenlänge in Nanometer angibt.

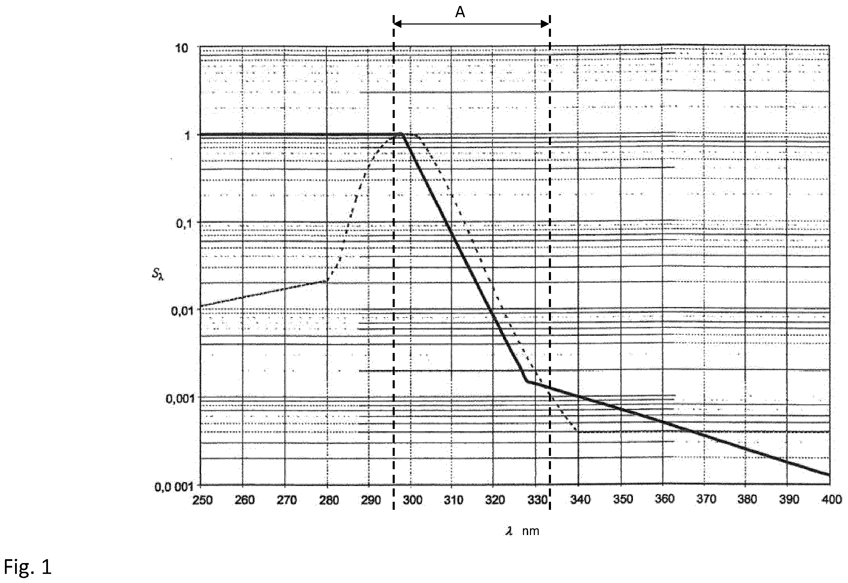

Die relative spektrale Wirksamkeit wird auch als Wirkungsfunktion bezeichnet und ist für das Erythem sowie den NMSC in Figur 1 aufgetragen über der Wellenlänge Lambda in Nanometern dargestellt. Die relative spektrale Wirksamkeit $S_\lambda$ ist einheitenlos und in Figur 1 logarithmisch aufgetragen. Die gestrichelte Kurve zeigt das Spektrum für NMSC, während die durchgezogene Linie das Spektrum für das Erythem zeigt. Es ist ersichtlich, dass insbesondere im Wellenlängenbereich zwischen ca. 296 nm bis 333 nm die Wirkungsfunktionen für das Erythem sowie für NMSC sehr nah beieinanderliegen, NMSC das Erythem sogar übersteigt.

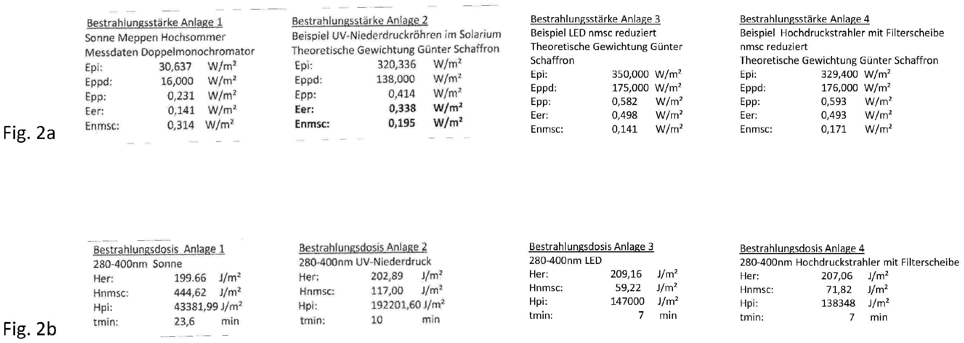

Figur 2a zeigt die Bestrahlungsstärken einer Anlage 1, nämlich Messdaten der Sonne in Meppen im Hochsommer erfasst über ein Doppel-Monochromator, die Bestrahlungsstärke einer Anlage 2, nämlich die theoretische Bestrahlungsstärke einer UV-Niederdruckröhre im Solarium, die Bestrahlungsstärke einer Anlage 3, nämlich die theoretische Bestrahlungsstärke einer UV-LED im Solarium, sowie die Bestrahlungsstärke einer Anlage 4, nämlich die theoretische Bestrahlungsstärke eines UV-Hochdruckstrahlers mit Filterscheibe im Solarium. Es ist für alle Anlagen die für die direkte Pigmentierung verantwortliche Bestrahlungsstärke $E_{pi}$, die für die indirekte Pigmentierung verantwortliche Bestrahlungsstärke $E_{pp}$, die erthemwirksame Bestrahlungsstärke $E_{er}$ sowie die NMSC-wirksame Bestrahlungsstärke $E_{NMSC}$

ersichtlich. Zudem ist eine sich ebenfalls auf bleibende Bräune beziehende Bestrahlungsstärke $E_{ppd}$ ersichtlich, die eine Kombination aus UVB und UVA Strahlung, wobei die indirekte Pigmentierung $E_{pp}$ vorwiegend im UVB-Bereich zu finden ist.

Wie zu erkennen, ist das Verhältnis von $E_{NMSC}$ zu $E_{er}$ bei Anlage 1, also bei der natürlichen Sonne im Hochsommer, bei > 2. Die erfindungsgemäße Anlage 2 betrifft ein Solarium mit UV-Niederdruckröhren und hat hingegen hat ein Verhältnis von $E_{NMSC}$ zu $E_{er}$ von nur 0,58. Zudem wurde $E_{NMSC}$ hier auf einen Wert von < 0,2 W/m$^2$ reduziert und ist damit geringer als der der natürlichen Sonne im Hochsommer. Der Wert für $E_{er}$ liegt hierbei noch unterhalb der in Europa zulässigen Höchstgrenze von 0,349 W/m$^2$. Die erfindungsgemäße Anlage 3 betrifft ein Solarium mit UV-LEDs und weist ein Verhältnis von $E_{NMSC}$ zu $E_{er}$ von 0,28 auf. Die erfindungsgemäße Anlage 4 betrifft ein Solarium mit UV-Hochdruckstrahlern mit Filterscheibe und weist ein Verhältnis von $E_{NMSC}$ zu $E_{er}$ von 0,35 auf. Bei den Anlagen 3 und 4 liegt der Wert für $E_{er}$ oberhalb von 0,349 W/m$^2$, jedoch noch unterhalb des beispielsweise in den USA zulässigen Höchstwerts.

Es wird somit bei den Anlagen 2 bis 4 das erfindungsgemäße Verhältnis von $E_{NMSC}/E_{ER} \leq 0,9$ erreicht. Dieses Verhältnis kann beispielsweise realisiert werden durch eine Ausbildung der Strahlungsquelle derart, dass in dem in Fig. 1 mit A gegenzeichneten Wellenlängenbereich zwischen ca. 296 nm bis ca. 333 nm, in dem die Wirkungsfunktion für NMSC über derjenigen für das Erythem liegt, keine Strahlung ausgesendet wird. Die Strahlungskörper der Strahlungsquelle können entsprechend ausgebildet sein. Grundsätzlich können aber auch Filter vorgesehen sein, die von den Strahlungskörpern in diesem Bereich ausgesendete Strahlung wegfiltern.

Die Anlage 2 enthält dabei theoretische Werte, die nach entsprechenden Vorgaben pro Nanometer Wellenlänge derart gewichtet wurden, dass das Verhältnis von $E_{NMSC}$ zu $E_{er} \leq 0,9$ ist. Die erythemwirksame Bestrahlungsstärke einer Strahlungsquelle ist die Grundlage zur Berechnung einer hauttypgerechten Besonnungszeit und Bestrahlungsdosis. Diese macht somit ein Sonnenbrandrisiko kalkulierbar. Bei der erfindungsgemäßen Anlage 2 wurde $E_{er}$ größer gewählt als bei der natürlichen Sonne, um innerhalb kürzerer Zeit dieselbe Erythem-Bestrahlungsdosis zu erhalten. Die hierbei vorgesehenen UV-Niederdruckröhren können durch entsprechende Wahl des Gasgemischs dazu ausgebildet sein, in dem Wellenlängenbereich zwischen ca. 296 nm bis ca. 333 nm keine Strahlung auszusenden, mit Ausnahme eines durch den sogenannten Quecksilberpeak bedingten Wellenlängenbereichs von ca. 311 nm bis 315 nm. Dieser Peak lässt sich üblicherweise nicht verhindern.

Bei der Anlage 3 kann durch entsprechende Ausbildung der LEDs, also durch entsprechende Wahl der Halbleiter, dass in dem Wellenlängenbereich zwischen ca. 296 nm bis 333 nm keine Strahlung ausgesendet wird. Bei der die Hochdruckstrahler aufweisenden Anlage 4 hingegen kann durch in dem Strahlengang angeordnete Filterscheiben erreicht werden, dass in dem Wellenlängenbereich zwischen ca. 296 nm bis ca. 333 nm keine Strahlung ausgesendet wird.

In Figur 2b ist die Bestrahlungsdosis für die Anlagen 1 bis 4 dargestellt, wobei hier die Bestrahlungsdosis für das Erythem $H_{er}$, die NMSC-Bestrahlungsdosis $H_{NMSC}$ sowie die Bestrahlungsdosis für die direkte Pigmentierung $H_{pi}$ dargestellt sind. Zudem ist die Bestrahlungszeit $t_{min}$ dargestellt, die so gewählt ist, dass für alle Anlagen in etwa dieselbe Erythem-Bestrahlungsdosis $H_{er}$ erfolgt.

Aus diesem Vergleich wird ersichtlich, dass bei gleicher Erythem-Bestrahlungsdosis $H_{er}$ bei der erfindungsgemäßen Anlagen 2 bis 4 eine wesentlich geringere NMSC Bestrahlungsdosis von gerade einmal 117 J/m$^2$ bzw. 59,22 J/m$^2$ bzw. 71,82 J/m$^2$ erreicht wird als bei Anlage 1, wo die NMSC-Bestrahlungsdosis mit 444,62 J/m$^2$ in etwa das Vier- bis Achtfache beträgt. Zudem wird mit den erfindungsgemäßen Anlagen eine wesentlich bessere direkte Pigmentierung erreicht, da die entsprechende Bestrahlungsdosis $H_{pi}$ größer ausfällt. Im Ergebnis zur natürlichen Sonne kann mit den erfindungsgemäßen Strahlungsquellen somit bei gleicher Erythem-Dosis eine stark verringerte NMSC-Bestrahlungsdosis sowie eine bessere direkte Pigmentierung erreicht werden. Folglich kann bei geringerem NMSC-Risiko trotzdem ein schönes Bräunungsergebnis erreicht werden.

**Patentansprüche**

1. Strahlungsquelle zur Abgabe von UV-Strahlung für ein Hautbestrahlungsgerät zur Bräunung der Haut, **gekennzeichnet durch** ein Verhältnis von NMSC-wirksamer Bestrahlungsstärke ($E_{NMSC}$) zu erythemwirksamer Bestrahlungsstärke ($E_{er}$) von kleiner oder gleich 0,9 ($E_{NMSC}/E_{er} \leq 0,9$).

2. Strahlungsquelle nach Anspruch 1, **dadurch gekennzeichnet, dass** das Verhältnis im Wesentlichen über den gesamten für die Hautbräunung relevanten Wellenlängenbereich kleiner oder gleich 0,9 ist.

3. Strahlungsquelle nach Anspruch 1 oder 2, **dadurch gekennzeichnet, dass** das Verhältnis im Wesentlichen über einen Wellenlängenbereich von ca. 280 nm bis ca. 340 nm kleiner oder gleich 0,9 ist.

4. Strahlungsquelle nach einem der vorhergehenden Ansprüche, **dadurch gekennzeichnet, dass** die Strahlungsquelle dazu ausgebildet ist, im Wesentlichen in einem Wellenlängenbereich von ca. 296 nm bis ca. 333 nm keine Strahlung auszusenden.

5. Strahlungsquelle nach einem der vorhergehenden Ansprüche, **dadurch gekennzeichnet, dass** die Strahlungsquelle dazu ausgebildet ist, ab einer Wellenlänge von 340 nm aufwärts keine Strahlung abzugeben.

6. Strahlungsquelle nach einem der vorhergehenden Ansprüche, **gekennzeichnet durch** ein Verhältnis von Vitamin-D3-wirksamer Bestrahlungsstärke ($E_{D3}$) zu erythemwirksamer Bestrahlungsstärke ($E_{er}$) von mindestens 0,14.

7. Strahlungsquelle nach einem der vorhergehenden Ansprüche, **dadurch gekennzeichnet, dass** die Vitamin-D3-wirksame Bestrahlungsstärke ($E_{D3}$) mindestens 0,03 W/m$^2$ beträgt.

8. Strahlungsquelle nach einem der vorhergehenden Ansprüche, **dadurch gekennzeichnet, dass** die Strahlungsquelle eine UV-Niederdrucklampe umfasst.

9. Strahlungsquelle nach Anspruch 8, **dadurch gekennzeichnet, dass** die UV-Niederdrucklampe dazu ausgebildet ist, in einem Wellenlängenbereich von ca. 296 nm bis ca. 333 nm nur in einem den Quecksilberpeak betreffenden Wellenlängenbereich Strahlung auszusenden.

10. Strahlungsquelle nach einem der vorhergehenden Ansprüche, **dadurch gekennzeichnet, dass** die Leuchtstofflampe einen UV-Hochdruckbrenner, insbesondere mit einer Filterscheibe, umfasst.

11. Strahlungsquelle nach einem der vorhergehenden Ansprüche, **dadurch gekennzeichnet, dass** die Strahlungsquelle eine UV-LED aufweist.

12. Strahlungsquelle nach einem der vorhergehenden Ansprüche, **gekennzeichnet durch** eine Bestrahlungsstärke für die direkte Pigmentierung ($E_{pi}$) von mindestens 180 Watt pro Quadratmeter, bevorzugt von mindestens 200 Watt pro Quadratmeter.

13. Hautbestrahlungsgerät zur Bräunung der Haut umfassend mindestens eine Strahlungsquelle nach einem der vorhergehenden Ansprüche.

14. Hautbestrahlungsgerät nach Anspruch 13, **dadurch gekennzeichnet, dass** das Hautbestrahlungsgerät ein Solarium ist, insbesondere eine Sonnenbank oder eine Sonnendusche.

Fig. 1

EP 4 088 779 A1

**Fig. 2a**

**Bestrahlungsstärke Anlage 1**
Sonne Meppen Hochsommer
Messdaten Doppelmonochromator

| | | |
|---|---|---|
| Epi: | 30,637 | W/m² |
| Eppd: | 16,000 | W/m² |
| Epp: | 0,231 | W/m² |
| Eer: | 0,141 | W/m² |
| Enmsc: | 0,314 | W/m² |

**Bestrahlungsstärke Anlage 2**
Beispiel UV-Niederdruckröhren im Solarium
Theoretische Gewichtung Günter Schaffron

| | | |
|---|---|---|
| Epi: | 320,336 | W/m² |
| Eppd: | 138,000 | W/m² |
| Epp: | 0,414 | W/m² |
| **Eer:** | **0,338** | **W/m²** |
| **Enmsc:** | **0,195** | **W/m²** |

**Bestrahlungsstärke Anlage 3**
Beispiel LED nmsc reduziert
Theoretische Gewichtung Günter
Schaffron

| | | |
|---|---|---|
| Epi: | 350,000 | W/m² |
| Eppd: | 175,000 | W/m² |
| Epp: | 0,582 | W/m² |
| Eer: | 0,498 | W/m² |
| Enmsc: | 0,141 | W/m² |

**Bestrahlungsstärke Anlage 4**
Beispiel Hochdruckstrahler mit Filterscheibe
nmsc reduziert
Theoretische Gewichtung Günter Schaffron

| | | |
|---|---|---|
| Epi: | 329,400 | W/m² |
| Eppd: | 176,000 | W/m² |
| Epp: | 0,593 | W/m² |
| Eer: | 0,493 | W/m² |
| Enmsc: | 0,171 | W/m² |

**Fig. 2b**

**Bestrahlungsdosis Anlage 1**
280-400nm Sonne

| | | |
|---|---|---|
| Her: | 199.66 | J/m² |
| Hnmsc: | 444,62 | J/m² |
| Hpi: | 43381,99 | J/m² |
| tmin: | 23,6 | min |

**Bestrahlungsdosis Anlage 2**
280-400nm UV-Niederdruck

| | | |
|---|---|---|
| Her: | 202,89 | J/m² |
| Hnmsc: | 117,00 | J/m² |
| Hpi: | 192201,60 | J/m² |
| tmin: | 10 | min |

**Bestrahlungsdosis Anlage 3**
280-400nm LED

| | | |
|---|---|---|
| Her: | 209,16 | J/m² |
| Hnmsc: | 59,22 | J/m² |
| Hpi: | 147000 | J/m² |
| tmin: | 7 | min |

**Bestrahlungsdosis Anlage 4**
280-400nm Hochdruckstrahler mit Filterscheibe

| | | |
|---|---|---|
| Her: | 207,06 | J/m² |
| Hnmsc: | 71,82 | J/m² |
| Hpi: | 138348 | J/m² |
| tmin: | 7 | min |

Europäisches Patentamt

European Patent Office

Office européen des brevets

## EUROPÄISCHER RECHERCHENBERICHT

Nummer der Anmeldung

EP 21 17 3008

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| T | Volkmer Beate ET AL: "Schutz des Menschen vor den Gefahren solarer UV- Strahlung und UV-Strahlung in Solarien", , 11. Februar 2016 (2016-02-11), Seiten 1-121, XP055852193, Bonn Gefunden im Internet: URL:https://www.ssk.de/SharedDocs/Beratung sergebnisse_PDF/2016/2016-02-11_Empf_UV-Sc hutz%20BA.pdf?__blob=publicationFile [gefunden am 2021-10-18] ----- | | INV. A61N5/06 |
| X | US 2013/172963 A1 (MOFFAT WILLIAM A [US]) 4. Juli 2013 (2013-07-04) * Absätze [0020] - [0033]; Ansprüche 1,2,8,16,17,19; Abbildungen 1A-C * ----- | 1-14 | |
| | | | RECHERCHIERTE SACHGEBIETE (IPC) A61N |

Der vorliegende Recherchenbericht wurde für alle Patentansprüche erstellt

| Recherchenort | Abschlußdatum der Recherche | Prüfer |
|---|---|---|
| München | 13. Dezember 2021 | Kajzar, Anna |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

...............................................................
& : Mitglied der gleichen Patentfamilie, übereinstimmendes Dokument

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 21 17 3008

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

13-12-2021

| Im Recherchenbericht angeführtes Patentdokument | Datum der Veröffentlichung | Mitglied(er) der Patentfamilie | | Datum der Veröffentlichung |
|---|---|---|---|---|
| US 2013172963 A1 | 04-07-2013 | AU | 2013206887 A1 | 14-08-2014 |
| | | AU | 2018200369 A1 | 08-02-2018 |
| | | CA | 2861620 A1 | 11-07-2013 |
| | | CN | 104168953 A | 26-11-2014 |
| | | CN | 107029357 A | 11-08-2017 |
| | | DK | 2800605 T3 | 29-01-2018 |
| | | EP | 2800605 A1 | 12-11-2014 |
| | | EP | 3345653 A1 | 11-07-2018 |
| | | ES | 2656893 T3 | 28-02-2018 |
| | | HK | 1203868 A1 | 06-11-2015 |
| | | JP | 6305933 B2 | 04-04-2018 |
| | | JP | 2015503406 A | 02-02-2015 |
| | | JP | 2017148533 A | 31-08-2017 |
| | | PL | 2800605 T3 | 30-03-2018 |
| | | PT | 2800605 T | 29-12-2017 |
| | | RU | 2014131906 A | 20-02-2016 |
| | | US | 2013172963 A1 | 04-07-2013 |
| | | US | 2019160303 A1 | 30-05-2019 |
| | | US | 2021260402 A1 | 26-08-2021 |
| | | WO | 2013103743 A1 | 11-07-2013 |

EPO FORM P0461

Für nähere Einzelheiten zu diesem Anhang : siehe Amtsblatt des Europäischen Patentamts, Nr.12/82